# EUROPEAN PATENT SPECIFICATION

(11) **EP 2 575 889 B1**
(45) Date of publication and mention of the grant of the patent: **22.04.2015**
(21) Application number: 11730742.1
(22) Date of filing: 31.05.2011
(51) Int. Cl.: A61K 47/10, A61K 47/12, A61K 9/08, A61K 31/436

(54) **STABLE PHARMACEUTICAL COMPOSITIONS OF RAPAMYCIN ESTERS**
STABILE PHARMAZEUTISCHE RAPAMYCINESTERZUSAMMENSETZUNGEN
COMPOSITIONS PHARMACEUTIQUES STABLES D'ESTERS DE RAPAMYCINE

(30) Priority: 02.06.2010 IN MU12762010
(43) Date of publication of application: 10.04.2013
(73) Proprietor: Fresenius Kabi Oncology Ltd, 110 066 New Delhi (IN)
(72) Inventor: KHATTAR, Dhiraj, Gurgaon 122 001, Haryana (IN); KHANNA, Rajesh, Gurgaon 122 001, Haryana (IN); SINGLA, Poonam, Gurgaon 122 001, Haryana (IN); YADAV, Abhilasha, Gurgaon 122 001, Haryana (IN); GUPTA, Vinay, Gurgaon 122 001, Haryana (IN); KINI, Rajesh, Gurgaon 122 001, Haryana (IN); DUBEY, Sushil, Kumar, Gurgaon 122 001, Haryana (IN)
(74) Representative: Jansen, Cornelis Marinus
(86) International application number: PCT/IB2011/001191
(87) International publication number: WO 2011/151704

(56) References cited:
- WO-A1-2004/011000
- WO-A1-2005/011688
- WO-A2-02/40000

## Description

### FIELD OF THE INVENTION

The present invention relates to stable pharmaceutical compositions of Rapamycin 42-ester with 3-hydroxy-2-(hydroxymethyl)-2-methylpropionic acid that are free of antioxidants.

### BACKGROUND OF THE INVENTION

Rapamycin is an immunosuppressive lactam macrolide and is also found to exhibit antitumor and antifungal activities. A number of derivatives of Rapamycin such as esters of Rapamycin are known to have antineoplastic activities.

Of significance is Rapamycin 42-ester with 3-hydroxy-2-(hydroxymethyl)-2-methylpropionic acid, generically known as Temsirolimus and represented by Formula-I shown below, is an antineoplastic agent indicated for the treatment of advanced renal cell carcinoma. This ester of Rapamycin has demonstrated significant inhibitory effects on tumor growth in both *in vitro* and *in vivo* models. Rapamycin 42-ester with 3-hydroxy-2-(hydroxymethyl)-2-methylpropionic acid was first disclosed by Skotnicki et al in US Patent No. 5,362,718. The preparation and use of hydroxyesters of Rapamycin, including Rapamycin 42-ester with 3-hydroxy-2-(hydroxymethyl)-2-methylpropionic acid is also disclosed in this Patent.

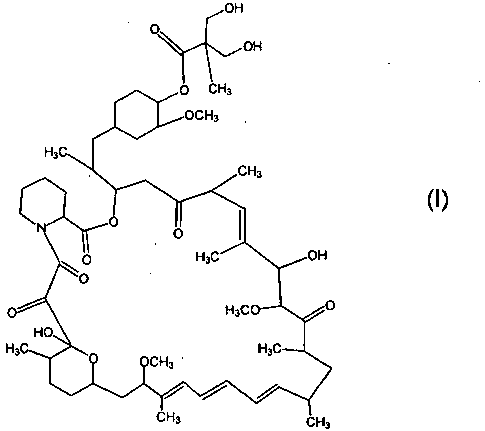

### Rapamycin 42-ester with 3-hydroxy-2-(hydroxymethyl)-2-methylpropionic acid (Temsirolimus)

With regards to its pharmacological activities, Rapamycin 42-ester with 3-hydroxy-2-(hydroxymethyl)-2-methylpropionic acid exhibits cytostatic properties and acts on the tumors by delaying their time of progression or their recurrence time. This ester of Rapamycin is considered to have a mechanism of action that is similar to that of the parent molecule Rapamycin.

Presently, Rapamycin 42-ester with 3-hydroxy-2-(hydroxymethyl)-2-methylpropionic acid (Temsirolimus) is marketed as an injectable formulation- under the brand name Torisel ™, which is available as a two vial presentation with the first vial comprising Temsirolimus as the active ingredient along with inactive ingredients like dehydrated alcohol (39.5% w/v), d,l-alpha-tocopherol (0.075% w/v), propylene glycol (50.3% w/v), and anhydrous citric acid (0.0025% w/v). The second vial contains a diluent for Torisel ™ injection containing inactive ingredients like polysorbate 80 (40.0% w/v), polyethylene glycol 400 (42.8% w/v), and dehydrated alcohol (19.9%w/v). The contents of the first and second vials are mixed with an infusion fluid and then administered to the patients in need thereof. Temsirolimus is most typically administered to patients by subcutaneous, intramuscular or intravenous route of which the more preferred routes are by a bolus I.V.injection, continuous I.V.infusion or I.V.infusion.

Thus, the marketed formulation may be said to contain Rapamycin 42-ester with 3-hydroxy-2-(hydroxymethyl)-2-methylpropionic acid dissolved in a mixture of solvents, which may be classified as "alcoholic solvents". These solvents such as dehydrated alcohol and propylene glycol are mainly responsible for solubilizing the drug, which is poorly soluble in aqueous solvents and has an aqueous solubility of less than 1 µg/ml. In addition to these solvents, the currently marketed formulation contains an antioxidant viz. d,1-alpha-tocopherol and also contains citric acid, which acts as an antioxidant as well as a chelating agent. Further, the second vial contains a mixture of diluents such as polyethylene glycol 400 and dehydrated alcohol along with a surfactant i.e. polysorbate 80. Hence, it may be said that the most critical element in the currently available pharmaceutical composition is the presence of antioxidants that prevents the oxidative degradation of the drug and provides stability to the formulation.

In addition to Rapamycin 42-ester with 3-hydroxy-2-(hydroxymethyl)-2-methylpropionic acid other commercially available Rapamycin esters such as Sirolimus (Rapamune™) Tablet is also formulated along with Antioxidants to form stable tablets that are not prone to oxidation. Hence, in general Rapamycin esters are prone to degradation by oxidation and hence are formulated with Antioxidants to develop stable pharmaceutical compositions.

From the various prior art disclosures it is evident that Rapamycin and its related compounds are susceptible to chemical instability during synthesis of the compounds or during their formulation as a dosage form. The chemical instability of Rapamycin esters is mainly attributed to their oxidative degradation or to cleavage of a lactone bond in the molecule, resulting in the opening of the ring and formation of a degradation product.

Hence, the main challenge lies in formulating a stable pharmaceutical composition of such Rapamycin esters that has the minimum concentration of oxidative degradation impurities. All the prior arts mainly provide solutions to the problem related to oxidative degradation of the drug by using an antioxidant in their formulation.
1. Rubino et al in US Application Publication No. 2004/0167152 discloses a formulation of Rapamycin 42-ester with 3-hydroxy-2-(hydroxymethyl)-2-methylpropionic acid utilizing an antioxidant as an essential ingredient in the formulation that reduces the amount of oxidative impurities in the composition, thus developing a stable pharmaceutical composition of Rapamycin 42-ester with 3-hydroxy-2-(hydroxymethyl)-2-methylpropionic acid. The formulation as disclosed in the Application may be said to cover the marketed Torisel™ formulation. The antioxidant as disclosed in the Application is further selected from the group comprising of citric acid, glycine, d,l-.alpha.-tocopherol, BHA, BHT, monothioglycerol, ascorbic acid, propyl gallate, and mixtures thereof. It is further disclosed in the Application that the antioxidant utilized in developing a stable pharmaceutical composition of Rapamycin 42-ester with 3-hydroxy-2-(hydroxymethyl)-2-methylpropionic acid is d,l-.alpha.-tocopherol and it is used in the concentration range of 0.01% to 0.1 %w/v with a preferred concentration of 0.075%w/v. Furthermore, it is disclosed that the antioxidant components of the formulation of the invention also exhibit chelating activity such as citric acid, acetic acid, and ascorbic acid. Of these acids citric acid is the most preferred acid. The Application also discloses a process for preparation of such a stable formulation. Hence, use of an antioxidant in the formulation is the key element in developing a stable pharmaceutical composition.
2. Rubino et al in another US Application Publication No. 2007/0142422, discloses a method for preparing a rapamycin composition having increased potency by selecting the active ingredient having less than 1.5% oxidative and hydrolytic rapamycin impurities and formulating the selected rapamycin with an antioxidant and optional excipients, wherein the antioxidant is alpha-tocopherol. The pH of the pharmaceutical composition developed is in the range of about 4 to about 6. Further, it is disclosed that the formulation developed may be administered by parenteral route as well as oral route. Therefore, in this Application also an antioxidant is utilized in the formulation to get a stable product.
3. Further, Rubino et al in US Application Publication No. 2005/0020615 discloses a stable lyophilized formulation of Rapamycin 42-ester with 3-hydroxy-2-(hydroxymethyl)-2-methylpropionic acid that utilizes an antioxidant in the formulation in a concentration ranging from 0.001% to 1% w/v wherein, the antioxidants are selected from the group consisting of BHT, BHA, alpha-tocopherol, ascorbic acid, erythorbic acid (0.1-1.0% w/v), ithiothreitol, dithioerythreitol, glutathione , ascorbyl palmitate, monothioglycerol, propylgallate, sodium bisulfite and sodium metabisulfite. Furthermore, it is disclosed that the antioxidant component of the formulation of the invention may also exhibit chelating activity such as citric acid, succinic acid, malic acid, maleic acid, malonic acid, glutaric acid and adipic acid. A number of examples of Rapamycin 42-ester with 3-hydroxy-2-(hydroxymethyl)-2-methylpropionic acid along with antioxidants have also been provided. Hence, as seen in this Application, antioxidants have been used in developing a stable lyophilized pharmaceutical composition of Rapamycin 42-ester with 3-hydroxy-2-(hydroxymethyl)-2-methylpropionic acid.
4. Ashraf et al in US Application Publication No. 2004/0077677 discloses a stable pharmaceutical composition of Rapamycin 42-ester with 3-hydroxy-2-(hydroxymethyl)-2-methylpropionic acid for oral administration comprising Rapamycin 42-ester with 3-hydroxy-2-(hydroxymethyl)-2-methylpropionic acid, a water soluble polymer, a surfactant, an antioxidant, and a pH modifying agent wherein the antioxidant is butylated hydroxyanisole and butylated hydroxytoluene. The Application further discloses a process for preparation of the pharmaceutical composition utilizing wet granulation method. Therefore, antioxidants have also been utilized for developing a stable oral formulation of Rapamycin 42-ester with 3-hydroxy-2-(hydroxymethyl)-2-methylpropionic acid.
5. Ashraf et al in U.S. Patent Application Publication No. 2005/0152983 discloses pharmaceutical compositions containing a stable and bioavailable form of micronized Rapamycin 42-ester with 3-hydroxy-2-(hydroxymethyl)-2-methylpropionic acid along with an antioxidant or a chelating agent, or mixtures thereof, in an immediate release dosage form for oral administration. The composition is in the form of a tablet or in filled capsules. Acceptable antioxidants include, but are not limited to, citric acid, d,l-.alpha.-tocopherol, butylated hydroxyanisol (BHA), butylated hydroxytoluene (BHT), monothioglycerol, ascorbic acid, propyl gallate, and mixtures thereof. Hence, antioxidants have been utilized along with Rapamycin 42-ester with 3-hydroxy-2-(hydroxymethyl)-2-methylpropionic acid in obtaining an immediate release dosage form for oral administration.
6. Zhu et al in US Patent No. 7,074,804 discloses an oral formulation of Rapamycin 42-ester with 3-hydroxy-2-(hydroxymethyl)-2-methylpropionic acid using wet granulation method, utilizing isomer C of the drug, a water soluble polymer, a pH modifying agent, a surfactant, and an antioxidant. The patent also provides an injectable formulation containing Rapamycin 42-ester with 3-hydroxy-2-(hydroxymethyl)-2-methylpropionic acid isomer C, a parenterally acceptable cosolvent, an antioxidant, a diluent solvent, and a surfactant. The patent further discloses that the stability of the isomer C in parenterally acceptable alcoholic cosolvents is enhanced by the addition of an antioxidant in the formulation, wherein the pharmaceutically acceptable antioxidants are selected from citric acid, d,l-α-tocopherol, BHA, BHT, monothioglycerol, ascorbic acid, propyl gallate, and mixtures thereof. It is further provided that the antioxidant component of the formulation of the invention also exhibits chelating activity. Examples of such chelating agents include citric acid, acetic acid, and ascorbic acid. Thus, the use of an antioxidant in the formulation is the most critical factor in obtaining a stable oral as well as a parenteral formulation.
7. Gu et al in US Patent No. 7 202 256 discloses that the stability of Rapamycin 42-ester with 3-hydroxy-2-(hydroxymethyl)-2-methylpropionic acid or proline-Rapamycin 42-ester with 3-hydroxy-2-(hydroxymethyl)-2-methylpropionic acid in parenterally acceptable alcoholic cosolvents is enhanced by the addition of an antioxidant to the formulation. Acceptable antioxidants include, but are not limited to, citric acid, d,l-α-tocopherol, BHA, BHT, monothioglycerol, ascorbic acid, propyl gallate, and mixtures thereof. The antioxidant component of the formulation of the invention also exhibits chelating activity. Examples of such chelating agents include, e.g., citric acid, acetic acid, and ascorbic acid (which may function as both a classic antioxidant and a chelating agent in the present formulations). Thus, this patent also talks of a stable pharmaceutical composition of Rapamycin 42-ester with 3-hydroxy-2-(hydroxymethyl)-2-methylpropionic acid utilizing antioxidant in the formulation.
8. Boni et al in US. Patent Application Publication No. 2006/0183766 discloses orally bioavailable Rapamycin 42-ester with 3-hydroxy-2-(hydroxymethyl)-2-methylpropionic acid formulations which advocates use of antioxidants in the manufacture of orally bioavailable Rapamycin 42-ester with 3-hydroxy-2-(hydroxymethyl)-2-methylpropionic acid formulations and the acceptable antioxidants include, but are not limited to, citric acid, d,l-alpha-tocopherol, butylated hydroxyanisol (BHA), butylated hydroxytoluene (BHT), monothioglycerol, ascorbic acid, propyl gallate, and mixtures thereof.
9. Navarro et al in US. Patent No. 7,297,703 discloses stabilization of another Rapamycin ester utilizing an antioxidant for preparation of a solid mixture for oral administration, wherein the antioxidant is selected from the group consisting of vitamin B, vitamin C, 2,6-di-tert-butyl-4-methylphenol (BHT), and combinations thereof.

Hence, from all the abovementioned prior art disclosures it is evident that the key element in all these pharmaceutical compositions of Rapamycin 42-ester with 3-hydroxy-2-(hydroxymethyl)-2-methylpropionic acid or other Rapamycin esters is that an antioxidant is invariably present in all these formulations. The main role of these antioxidants is to prevent the oxidative degradation of the Rapamycin esters and provide chemical stability to the various parenteral as well as oral formulations. This clearly indicates that till date a stable pharmaceutical composition of Rapamycin 42-ester with 3-hydroxy-2-(hydroxymethyl)-2-methylpropionic acid has always been obtained by the addition of an antioxidant in the formulation. Hence, the presence of an antioxidant in the any dosage form has been found to be the quintessential element in the formulation of a stable pharmaceutical composition of Rapamycin 42-ester with 3-hydroxy-2-(hydroxymethyl)-2-methylpropionic acid as it reduces the oxidative degradation of the drug and provides stability to the formulation.

However, a point of mention is that such agents as antioxidants and chelating agents-qualify as extraneous agents in the formulation of any pharmaceutical compositions. It may further be mentioned that Health Authorities all over the world are very concerned about the level of such extraneous agents as antioxidants, chelating agents and preservatives in the pharmaceutical compositions. As a consequence, regulatory approval norms today are very stringent about the nature and level of extraneous agents present in any drug product. In view of this, the range or freedom available to experiment with various extraneous agents such as antioxidants is minimum and they cannot be utilized beyond a limited amount. The presence of any unapproved range of excipients in the pharmaceutical formulations may have harmful effects on the patients and hence, such formulations are not acceptable to the Health Authorities, even if such formulations are stable. Keeping the aforementioned limitations in mind it is essential for the formulators to develop a pharmaceutical composition that is stable and contains any extraneous agents in the formulation in quantities that fall within the regulatory guidelines and do not have any negative impacts on the health of the patients.

Hence, there is a need to develop pharmaceutical compositions of Rapamycin 42-ester with 3-hydroxy-2-(hydroxymethyl)-2-methylpropionic acid that are free of any extraneous agents such as antioxidants and are also stable such that such formulations are more patient compliant.

Against this backdrop the inventors of the present application have surprisingly found that stable pharmaceutical compositions of Rapamycin 42-ester with 3-hydroxy-2-(hydroxymethyl)-2-methylpropionic acid may be developed without the use of extraneous agents such as antioxidants in the composition. It has further been found that such pharmaceutical compositions show comparable if not better stability than the currently marketed Torisel^{™} formulation and this forms the basis of the present application.

### SUMMARY OF THE INVENTION

The present invention provides stable pharmaceutical compositions of Rapamycin 42-ester with 3-hydroxy-2-(hydroxymethyl)-2-methylpropionic acid that are free of antioxidants.

The pharmaceutical compositions thus developed show comparable if not better stability than the currently marketed Torisel^{™} formulation that contains an antioxidant as the key component in the composition to reduce the amount of oxidative degradation and form a stable composition. As exemplified in the below-mentioned examples 1-7, the comparative stability profile of the compositions of the present invention with a composition that is similar to the marketed Torisel^{™} formulation as shown in Tables 1B-7B show that all the compositions of the present invention show comparable if not better stability than the currently marketed Torisel^{™} formulation when the same are stored at 40°C, 25°C and 2-8°C for a period ranging from seven days to six months.

One aspect of the present invention provides stable pharmaceutical compositions of Rapamycin 42-ester with 3-hydroxy-2-(hydroxy methyl)-2-methylpropionic acid that may be suitable for parenteral administration. Such parenteral formulations contain Rapamycin 42-ester with 3-hydroxy-2-(hydroxymethyl)-2-methylpropionic acid dissolved in pharmaceutically acceptable solvents, wherein the solvents are alcoholic solvents. The pharmaceutical compositions further comprise lactic acid and a surfactant. The parenteral formulations of the present invention may be further provided as a freeze dried formulation or as a ready-to-use pharmaceutical composition. Such parenteral pharmaceutical compositions do not contain antioxidants and are found to exhibit comparable if not better stability than the currently available marketed formulation of Rapamycin 42-ester with 3-hydroxy-2-(hydroxymethyl)-2-methylpropionic acid.

Another aspect of the present invention provides a two vial parenteral pharmaceutical composition of Rapamycin 42-ester with 3-hydroxy-2-(hydroxymethyl)-2-methylpropionic acid, in which the first vial comprises the drug dissolved in a solvent mixture, wherein the solvents are selected from "alcoholic solvents" comprising of ethanol, propylene glycol and polyethylene glycol. In addition, the composition further comprises lactic acid and a surfactant. The contents of the second vial comprise of diluents and may optionally contain a surfactant. The contents of the two vials are mixed together and then added to the infusion fluid before administration to the patients in need thereof by intravenous infusion. The pharmaceutical composition thus provided does not contain any antioxidant and exhibits comparable if not better stability than the currently available marketed formulation of Rapamycin 42-ester with 3-hydroxy-2-(hydroxymethyl)-2-methylpropionic acid.

Another aspect of the present invention provides a single vial parenteral pharmaceutical composition of Rapamycin 42-ester with 3-hydroxy-2-(hydroxymethyl)-2-methylpropionic acid, in which the drug is dissolved in a solvent mixture, wherein the solvents are selected from "alcoholic solvents" comprising of ethanol, propylene glycol and polyethylene glycol. In addition, the composition comprises lactic acid and a surfactant. During administration to the patients, the entire contents of the vial are added to the infusion fluid and then administered to the patients in need thereof by intravenous infusion. The pharmaceutical composition thus provided does not contain any antioxidant and exhibits comparable if not better stability than the currently available marketed formulation of Rapamycin 42-ester with 3-hydroxy-2-(hydroxymethyl)-2-methylpropionic acid.

The fourth aspect of the invention provides solid pharmaceutical compositions of Rapamycin 42-ester with 3-hydroxy-2-(hydroxymethyl)-2-methylpropionic acid for oral administration. The solid pharmaceutical compositions comprise Rapamycin 42-ester with 3-hydroxy-2-(hydroxymethyl)-2-methylpropionic acid along with pharmaceutically acceptable excipients such as binders, fillers, disintegrants and lubricants. In addition to these ingredients, the formulations also contain a surfactant and lactic acid. The solid dosage form thus provided is prepared from granules that are obtained by wet granulation utilizing a solvent system comprising water and an alcohol, with ethanol being the preferred alcoholic component. Other alcohols that may also be utilized in the pharmaceutical compositions are propylene glycol and polyethylene glycol. An important aspect of such pharmaceutical compositions is that they do not contain any antioxidants and are found to be as stable as the currently available marketed formulation.

Another aspect of the present invention provides a process for the preparation of stable pharmaceutical compositions of Rapamycin 42-ester with 3-hydroxy-2-(hydroxymethyl)-2-methylpropionic acid , wherein, the alcoholic solvents are first added to the formulation vessel followed by the addition of Rapamycin 42-ester with 3-hydroxy-2-(hydroxymethyl)-2-methylpropionic acid An important aspect of the preparation of the stable composition is nitrogen purging that is started through the sparger into the formulation vessel. In addition nitrogen atmosphere is maintained by flushing nitrogen in the headspace throughout the preparation process. For a single vial the diluents mix is added in the same vessel and for the two vial formulation the diluents mix is added to another vessel: The final volume is made up with the alcoholic solvent viz. dehydrated alcohol. The contents are then filled into type I clear glass vial with nitrogen. Before administration to the patients the contents of the vials are added into diluting fluids such as 0.9% sodium chloride injection, 5% dextrose injection, and other commonly used intravenous infusion solutions prior to administration to the patients by intravenous infusion.

Hence, the most important aspect in all the abovementioned pharmaceutical compositions of the present invention is that all these formulations of Rapamycin 42-ester with 3-hydroxy-2-(hydroxymethyl)-2-methylpropionic acid are free of antioxidants and are found to exhibit comparable if not better stability than the currently available marketed formulation of Rapamycin 42-ester with 3-hydroxy-2-(hydroxymethyl)-2-methylpropionic acid (Torisel™).

### DETAILED DESCRIPTION OF THE INVENTION

The present invention is directed to stable pharmaceutical compositions Rapamycin 42-ester with 3-hydroxy-2-(hydroxymethyl)-2-methylpropionic acid that are free of antioxidants.

The pharmaceutical compositions thus prepared show comparable if not better stability than the marketed Torisel™ formulation that contains an antioxidant to reduce the amount of oxidative degradation in the formulation.

The parenteral formulations of the present invention may be provided as a lyophilized formulation as well as a ready-to-use solution that are suitable for parenteral administration. This formulation may further be presented as a two vial presentation or as a single vial presentation having Rapamycin 42-ester with 3-hydroxy-2-(hydroxymethyl)-2-methylpropionic acid concentrations in the range of 1 to 50 mg/ml of which the preferred concentration range lies between 10 to 25 mg/ml. These pharmaceutical compositions may be administered via intravenous infusion to treat patients suffering from advanced renal cell carcinoma, which is the approved indication of Rapamycin 42-ester with 3-hydroxy-2-(hydroxymethyl)-2-methylpropionic acid.

The parenteral formulations thus provided comprise Rapamycin 42-ester with 3-hydroxy-2-(hydroxymethyl)-2-methylpropionic acid solvated in a non-aqueous parenterally acceptable solvent, wherein the solvent is an alcoholic solvent containing one or more alcohols as the alcoholic solvent component of the formulation. These solvents are selected from the group of solvents comprising ethanol, propylene glycol, polyethylene glycol 300, polyethylene glycol 400, polyethylene glycol 600 or polyethylene glycol 1000. These alcoholic solvents are particularly desirable because degradation via oxidation and lactone cleavage occurs to a lower extent in the presence of these solvents. Of these alcoholic solvents, the most preferred solvents are ethanol, propylene glycol and polyethylene glycol 400.

In addition, the pharmaceutical compositions of the present invention contain lactic acid. It is believed that adding lactic acid to the formulation to maintain a slightly acidic pH (e.g., within pH 3-5) facilitates ready dissolution of Rapamycin 42-ester with 3-hydroxy-2-(hydroxymethyl)-2-methylpropionic acid in the solvent and enhances long-term stability of the formulation. The presence of lactic acid in the formulation maintains the pH of the solution in the range of 3.0 to 5.0 and more preferably in the range of 4.0-4.5.

The pharmaceutical formulations of the present invention also include a parenterally acceptable surfactant wherein the surfactant is selected from the group comprising of polysorbate 20, polysorbate 80, a bile acid, lecithin, an ethoxylated vegetable oil, vitamin E tocopherol propylene glycol succinate, or polyoxyethylene-polyoxypropylene block copolymers. Of these surfactants, the more preferred surfactants are polysorbate 20 and polysorbate 80. Of these, polysorbate 80 is the most preferred parenterally acceptable surfactant that is used in the pharmaceutical compositions of the present invention.

As provided in Examples 1-5, the parenteral pharmaceutical compositions may also be provided as a two vial presentation, wherein one vial contains the Rapamycin 42-ester with 3-hydroxy-2-(hydroxymethyl)-2-methylpropionic acid dissolved in a parenterally acceptable solvent mixture comprising of ethanol, propylene glycol and polyethylene glycol. In addition, the vial also contains lactic acid and a surfactant as mentioned hereinbefore. The second vial contains a diluent mixture that contains a diluent solvent and optionally a suitable surfactant that is selected from the abovementioned group of surfactants. During administration to the patients in need thereof, the contents of the two vials are mixed together and then added to the sterile infusion solutions such as 0.9% sodium chloride injection, 5% dextrose injection, and other commonly used intravenous infusion solutions prior to administration to the patients by intravenous infusion.

In another aspect, as provided in Examples 6-7, the parenteral formulations of the present invention may be prepared and presented as a single vial formulation, wherein the drug is dissolved in a parenterally acceptable solvent mixture comprising of ethanol, propylene glycol and polyethylene glycol. In addition to this lacic acid and a surfactant, as mentioned hereinbefore are present in the vial. During administration to the patients, the entire contents of the vial are added to the infusion fluids such as 0.9% sodium chloride injection, 5% dextrose injection, and other commonly used intravenous infusion solutions and then administered to the patients in need thereof by intravenous infusion.

The pharmaceutical compositions thus provided in Examples 1-7 are free of antioxidants in the formulation and exhibit comparable and even better stability profile than the currently marketed formulation of the Rapamycin ester. As may be seen from the stability profiles presented in Tables 1 B, 2B, 3B, 4B, 5B, 6B and 7B, all the formulations of the present invention are found to exhibit comparable and at times even better stability than the currently marketed formulation of Rapamycin 42-ester with 3-hydroxy-2-(hydroxymethyl)-2-methylpropionic acid when the same are stored at 40°C, 25°C and 2-8°C for a period of seven days to six months.

The present invention also provides solid pharmaceutical compositions of Rapamycin 42-ester with 3-hydroxy-2-(hydroxymethyl)-2-methylpropionic acid that are suitable for oral administration. These solid pharmaceutical compositions contain Rapamycin 42-ester with 3-hydroxy-2-(hydroxymethyl)-2-methylpropionic acid along with pharmaceutically acceptable excipients such as binders, fillers, disintegrants and lubricants that are selected from the group comprising of sucrose, lactose, microcrystalline cellulose, croscarmellose sodium, magnesium stearate, gum acacia, cholesterol, tragacanth, stearic acid, gelatin, casein, lecithin (phosphatides), carboxymethylcellulose calcium, carboxymethylcellulose sodium, methylcellulose, hydroxhylcellulose,hydroxypropylcellulose, hydroxypropylmethylcellutose phthalate, noncrystalline cellulose, cetostearyl alcohol, cetyl alcohol, cetyl esters wax, dextrates, dextrin, lactose, dextrose, glyceryl monooleate, glyceryl monostearate, glyceryl palmitostearate, polyoxhylene alkyl ethers, polhylene glycols, polyoxhylene castor oil derivatives, polyoxhylene stearates, and polyvinyl alcohol. In addition to these ingredients, the formulation also contains a surfactant and lactic acid as mentioned hereinbefore. The solid dosage forms thus provided are prepared by wet granulation, wherein the granules are obtained utilizing a solvent system comprising water and an alcohol, with ethanol being the preferred alcoholic component. Other alcohols that may also be utilized as a solvent are propylene glycol and polyethylene glycol. An important aspect of such pharmaceutical compositions are that they do not contain any antioxidants and are found to have similar if not better stability than the currently marketed formulation.

Another aspect of the present invention provides a process for the preparation of the two vial pharmaceutical composition which is as provided below:

### Preparation of the contents of the first vial:

First of all the temperature of the formulation vessel is maintained at 2° - 8°C. To this is added 90% of the required quantity of the alcoholic solvents viz. dehydrated alcohol and propylene glycol. Polysorbate 80 is added to the formulation vessel. Further lactic acid stock solution is prepared in ethanol which is added into the formulation vessel under constant stirring. An important aspect of the preparation is nitrogen purging that is started through the sparger into the formulation vessel. Also nitrogen atmosphere is maintained by flushing nitrogen in the headspace throughout the process. At this stage Rapamycin 42-ester with 3-hydroxy-2-(hydroxymethyl)-2-methylpropionic acid is slowly added to the formulation vessel under constant stirring and the volume is made up to 100% with dehydrated alcohol. This solution is filtered through a suitable 0.22µ sterilizing grade filter under nitrogen and filled in type I clear glass vial with nitrogen flushing.

### Preparation of the contents of the second vial:

In a formulation vessel, required quantity of alcoholic solvents viz. polyethylene glycol 400 is added and stirring continued. To this is added required quantity of propylene glycol and optionally polysorbate 80 under continuous stirring. Finally, required quantity of dehydrated alcohol is added to the formulation vessel under continuous stirring until a uniform solution is obtained. Nitrogen gas is purged through a sparger into the formulation vessel. The bulk is filtered through suitable 0.22µ sterilizing grade filter under nitrogen and filled in type I clear glass vial with nitrogen flushing.

Finally, the contents of the two vials are added to a diluting fluid comprising sterile infusion solutions such as 0.9% sodium chloride injection, 5% dextrose injection, and other commonly used intravenous infusion solutions and then administered to the patients in need thereof.

In another aspect there is provided a process for the preparation of a single vial pharmaceutical composition of Rapamycin 42-ester with 3-hydroxy-2-(hydroxymethyl)-2-methylpropionic acid , wherein in the first step the temperature of formulation vessel is maintained at 2° - 8°C. Then 80% of the required quantity of dehydrated alcohol is taken into the formulation vessel. This is followed by the addition of lactic acid stock solution in ethanol under constant stirring. Nitrogen purging is started through the sparger into the formulation vessel. Also nitrogen atmosphere is maintained by flushing nitrogen in the headspace throughout the process. To this solution Rapamycin 42-ester with 3-hydroxy-2-(hydroxymethyl)-2-methylpropionic acid is slowly added under constant stirring. After drug dissolution, required quantity of the alcoholic solvents viz. propylene glycol, polyethylene glycol 400 and optionally polysorbate 80 is added to the formulation vessel under constant stirring. The volume is made up to 100% of the batch size with dehydrated alcohol. This solution is filtered through suitable 0.22µ sterilizing grade filter under nitrogen and filled in type I clear glass vial with nitrogen flushing.

Finally, the contents of the vial is added into a diluting fluid comprising sterile infusion solutions such as 0.9% sodium chloride injection,5% dextrose injection, and other commonly used intravenous infusion solutions and then administered to the patients in need thereof.

The major difference between the pharmaceutical compositions of the present invention and the marketed formulation lies in the absence of antioxidants in the formulations of the present invention. However, these formulations have been found to be as stable as the marketed formulation and have also been found to exhibit better stability than the marketed formulation.

The present invention is further illustrated by way of the following examples, which in no way should be construed as limiting the scope of the invention.

### EXAMPLES

The pharmaceutical compositions of the present invention as mentioned above may be provided as a single vial or a two vial composition. Examples 1-5 provide two vial compositions of Rapamycin 42-ester with 3-hydroxy-2-(hydroxymethyl)-2-methylpropionic acid that are essentially free of antioxidants. Examples 6-7 provide single vial compositions of Rapamycin 42-ester with 3-hydroxy-2-(hydroxymethyl)-2-methylpropionic acid that are essentially free of antioxidants. Further a comparative stability profile is provided in Tables 1 B, 2B, 3B, 4B, 5B, 6B and 7B of a composition that is similar to the marketed Torisel™ composition and essentially contains Rapamycin 42-ester with 3-hydroxy-2-(hydroxymethyl)-2-methylpropionic acid along with an antioxidant and acid (citric acid) with the stability profile of the compositions of the present invention that are essentially free of any antioxidants. From the stability data provided in these Tables, it is evident that the pharmaceutical compositions of the present invention exhibit comparable and sometimes even better stability profile than the pharmaceutical composition that is similar to the marketed composition when they are stored under similar storage conditions for the same time period.

Due to the unavailability of Torisel ^{™} product, the inventors of the present invention have prepared a pharmaceutical composition that is exactly similar to the Torisel^{™} composition which is as provided below:

Rapamycin 42-ester with 3-hydroxy-2-(hydroxymethyl)-2-methylpropionic acid dissolved in a mixture of dehydrated alcohol (39.5% w/v), d,l-alpha-tocopherol (0.075% w/v), propylene glycol (50.3% w/v), and anhydrous citric acid (0.0025% w/v). The diluent containing polysorbate 80 (40.0% w/v), polyethylene glycol 400 (42.8% w/v), and dehydrated alcohol (19.9%w/v). This pharmaceutical composition is similar to the marketed composition.

Various embodiments of the pharmaceutical compositions according to the present invention were prepared and studied for their stability and impurity profile when stored under various accelerated and real time stability conditions. A comparison of the stability profile of the pharmaceutical compositions prepared as per the present invention with the composition that is similar to the marketed composition is also provided in Tables 1 B-7B as illustrated below:

**Comparative example 1:** The pharmaceutical composition provided in this example is a two vial composition of Rapamycin 42-ester with 3-hydroxy-2-(hydroxymethyl)-2-methylpropionic acid free of any antioxidant and chelating agent (acid).The composition comprises Rapamycin 42-ester with 3-hydroxy-2-(hydroxymethyl)-2-methylpropionic acid dissolved in a mixture of alcoholic solvents viz. propylene glycol and dehydrated alcohol in one vial and the second vial contains the diluents which is a mixture of polysorbate 80, polyethylene glycol 400 and dehydrated alcohol. The unit composition formula of the pharmaceutical composition prepared by the present inventors is provided in Table-IA.

**Table-1A: unit composition formula of a two vial formulation of Rapamycin 42-ester with hydroxy-2-(hydroxymethyl)-2-methylpropionic. acid that is free of antioxidant and acid**

| **Component** | **Quantity per MI** |
|---|---|
| **Vial 1 (Drug Concentrate):** | 25 mg |
| Rapamycin 42-ester with 3-hydroxy-2-(hydroxymethyl)-2-methylpropionic acid | |
| Propylene glycol | 50.3% w/v |
| Dehydrated alcohol | 39.5% w/v |
| **Vial 2 (Diluent):** | 40.0% w/v |
| Polysorbate 80 | |
| Polyethylene glycol 400 | 42.8% w/v |
| Dehydrated alcohol | 19.9% w/v |

**Table-1B: Comparative stability profile of the composition that is similar to the marketed composition with the composition of present invention**

| **Composition** | **Condition** | **pH** | **Total impurities** |
|---|---|---|---|
| Composition similar to the marketed composition | Initial | 4.40 | 0.93 |
| | 40°C/7Days | 4.30 | 5.35 |
| | 25°C/ Month | 4.38 | 5.33 |
| | 25°C/ 2 Months | 4.36 | 7.31 |
| | 25°C/ 3 Months | 4.36 | 8.76 |
| | 25°C/6 Months | 4.34 | 9.82 |
| | 2-8°C/ Month | 4.36 | 1.98 |
| | 2-8°C/ 2 Months | 4.40 | 2.79 |
| | 2-8°C/3 Months | 4.43 | 3.31 |
| | 2-8°C/ 6 Months | 4.46 | 4.02 |
| Formulation of the present invention | Initial | 5.86 | 0.87 |
| | 40°C/7Days | 5.34 | 8.09 |
| | 25°C/ Month | 5.42 | 8.04 |
| | 25°C/ 2 Months | 5.42 | 8.27 |
| | 25°C/ 3 Months | 5.49 | 8.77 |
| | 25°C/ 6 Months | 5.50 | 8.72 |
| | 2-8°C/1 Month | 5.39 | 2.12 |
| | 2-8°C/ 2 Months | 5.55 | 3.31 |
| | 2-8°C/3 Months | 5.42 | 3.29 |
| | 2-8°C/ 6 Months | 5.42 | 3.93 |

The comparative stability profile of the pharmaceutical composition prepared from the formulation of the present invention and a pharmaceutical composition similar to the marketed composition shows that under stress conditions at 40° C, the impurity profile of the composition of the present invention is higher than the impurity profile of the composition that is similar to the marketed formulation under the same conditions over a period of seven days. When both the formulations were kept at 25°C for a period of three months and six months then the formulation of the present invention may be said to exhibit a comparable and even slightly better stability profile in terms of the total impurity profile as compared to the formulation that is similar to the marketed formulation. When stored at 2-8°C for a period of three and six months the two formulations are found to exhibit comparable stability.

Hence, the abovementioned pharmaceutical composition of the present invention shows relatively similar stability as the composition that is similar to the marketed formulation of Rapamycin 42-ester with 3-hydroxy-2-(hydroxymethyl)-2-methylpropionic acid.

**Example 2:** The pharmaceutical composition provided in this example is a two vial formulation of Rapamycin 42-ester with 3-hydroxy-2-(hydroxymethyl)-2-methylpropionic acid free of any antioxidant and contains lactic acid in place of citric acid. The composition comprises Rapamycin 42-ester with 3-hydroxy-2-(hydroxymethyl)-2-methylpropionic acid dissolved in a mixture of alcoholic solvents viz. propylene glycol and dehydrated alcohol along with lactic acid in one vial and the second vial contains the diluents which is a mixture of polysorbate 80, polyethylene glycol 400 and dehydrated alcohol. The unit composition formula of the pharmaceutical composition prepared by the present inventors is provided in Table-IIA.

**Table- 2 A: Unit composition formula of a two vial formulation of Rapamycin 42-ester with 3-hydroxy-2-(hydroxymethyl)-2-methylpropionic acid that is free of antioxidant and contains lactic acid**

| **Component** | **Quantity per mL** |
|---|---|
| **Vial 1 (Drug Concentrate):** | 25 mg |
| Rapamycin 42-ester with 3-hydroxy-2-(hydroxymethyl)-2-methylpropionic acid | |
| Propylene glycol | 50.3% w/v |
| Lactic acid | 0.025 mg |
| Dehydrated alcohol | 39.5% w/v |
| **Vial 2 (Diluent):** | 40.0% w/v |
| Polysorbate 80 | |
| Polyethylene glycol 400 | 42.8% w/v |
| Dehydrated alcohol | 19.9% w/v |

**Table- 2 B: Comparative stability profile of the composition that is similar to the marketed composition with the composition of the present invention**

| **Formulation** | **Condition** | **pH** | **Total impurities** |
|---|---|---|---|
| Composition similar to the marketed composition | Initial | 4.40 | 0.93 |
| | 40°C/7Days | 4.30 | 5.35 |
| | 25°C/ Month | 4.38 | 5.33 |
| | 25°C/ 2 Months | 4.36 | 7.31 |
| | 25°C/ 3 Months | 4.36 | 8.76 |
| | 25°C/ 6 Months | 4.34 | 9.82 |
| | 2-8°C/ Month | 4.36 | 1.98 |
| | 2-8°C/ 2 Months | 4.40 | 2.79 |
| | 2-8°C/3 Months | 4.43 | 3.31 |
| | 2-8°C/ 6 Months | 4.46 | 4.02 |
| Formulation of the present invention | Initial | 5.19 | 0.64 |
| | 40°C/7Days | 5.16 | 3.01 |
| | 25°C/ Month | 5.17 | 5.13 |
| | 25°C/2 Months | 5.18 | 7.12 |
| | 25°C/ 3 Months | 5.18 | 8.11 |
| | 2-8°C/1 Month | 5.14 | 1.93 |
| | 2-8°C/2 Months | 5.12 | 2.87 |
| | 2-8°C/3 Months | 5.12 | 3.37 |
| | 2-8°C/ 6 Months | 5.09 | 3.91 |

The comparative stability profile of the pharmaceutical composition prepared from the formulation protocol of the present invention and a formulation that is similar to the marketed composition shows that under stress conditions at 40° C, the impurity profile of the composition of the present invention is quite similar to the impurity profile of the marketed formulation kept under similar storage conditions for a period of seven days. However, under other storage conditions such as 25°C for a period of three months and six months, the formulation of the present invention shows a much better stability profile as compared to the formulation of the present invention. Similarly, at 2-8°C the formulation of the present invention shows a better stability profile as compared to the formulation that is similar to the marketed formulation when the two are stored over a period of three months and six months respectively.

Hence, the abovementioned pharmaceutical composition of the present invention shows relatively similar and even better stability profile than the composition that is similar to the marketed formulation of Rapamycin 42-ester with 3-hydroxy-2-(hydroxymethyl)-2-methylpropionic acid.

**Comparative example 3:** The pharmaceutical composition provided in this example is a two vial formulation of Rapamycin 42-ester with 3-hydroxy-2-(hydroxymethyl)-2-methylpropionic acid free of any antioxidant and acid. The composition comprises Rapamycin 42-ester with 3-hydroxy-2-(hydroxymethyl)-2-methylpropionic acid dissolved in polysorbate 80(surfactant) in one vial and the second vial contains the diluents which is a mixture of propylene glycol, polyethylene glycol 400 and dehydrated alcohol. The unit composition formula of the pharmaceutical composition prepared by the present inventors is provided in Table-IIIA.

**Table- 3A: Unit composition formula of a two vial formulation of Rapamycin 42-ester with 3-hydroxy-2-(hydroxymethyl)-2-methylpropionic acid that is free of any antioxidant and contains Rapamycin 42-ester with 3-hydroxy-2-(hydroxymethyl)-2-methylpropionic acid dissolved in a surfactant (polysorbate 80)**

| **Component** | **Quantity per mL** |
|---|---|
| **Vial 1 (Drug Concentrate):** | 25 mg |
| Rapamycin 42-ester with 3-hydroxy-2-(hydroxymethyl)-2-methylpropionic acid | |
| Polysorbate 80 | q.s. to 1 ml |
| Vial 2 (Diluent): | 42.8% w/v |
| Polyethylene glycol 400 | |
| Propylene glycol | 33.5% w/v |
| Dehydrated alcohol | 46.23% w/v |

**Table-3 B : Comparative stability profile of the composition that is similar to the marketed composition with the composition of the present invention**

| **Formulation** | **Condition** | **pH** | **Total impurities** |
|---|---|---|---|
| Composition similar to the marketed composition | Initial | 4.40 | 0.93 |
| | 40°C/7Days | 4.30 | 5.35 |
| | 25°C/ 3 Months | 4.36 | 8.76 |
| | 2-8°C/3 Months | 4.43 | 3.31 |
| Formulation of the present invention | Initial | 5.20 | 1.56 |
| | 40°C/7Days | 5.20 | 2.48 |
| | 25°C/3 Months | 5.30 | 4.28 |
| | 2-8°C/3 Months | 5.35 | 2.13 |

The comparative stability profile of the pharmaceutical composition prepared from the formulation protocol of the present invention and a formulation that is similar to the marketed composition shows that under stress conditions at 40° C, the impurity profile of the composition of the present invention is substantially less than the impurity profile of the marketed formulation kept under similar storage conditions for a period of seven days. Under other storage conditions such as 25°C for a period of three months the formulation of the present invention shows a much better stability profile as compared to the formulation of the present invention. Similarly, at 2-8°C the formulation of the present invention shows a better stability profile as compared to the formulation that is similar to the marketed formulation when the two are stored over a period of three months.

Hence, the abovementioned pharmaceutical composition of the present invention can be said to be more stable than the composition that is similar to the marketed formulation of Rapamycin 42-ester with 3-hydroxy-2-(hydroxymethyl)-2-methylpropionic acid.

**Example 4:** The pharmaceutical composition provided in this example is a two vial formulation of Rapamycin 42-ester with 3-hydroxy-2-(hydroxymethyl)-2-methylpropionic acid free of any antioxidant. The composition comprises Rapamycin 42-ester with 3-hydroxy-2-(hydroxymethyl)-2-methylpropionic acid dissolved in polysorbate 80(surfactant) along with lactic acid in one vial and the second vial contains the diluents which is a mixture of propylene glycol, polyethylene glycol 400 and dehydrated alcohol. The unit composition Formula of the pharmaceutical composition prepared by the present inventors is provided in Table-IVA.

**Table- 4 A: Two vial formulation of Rapamycin 42-ester with 3-hydroxy-2-(hydroxymethyl)-2-methylpropionic acid free of antioxidant and containing the drug dissolved in a Surfactant (polysorbate 80) along with an acid (lactic acid)**

| **Component** | **Quantity per mL** |
|---|---|
| **Vial 1 (Drug Concentrate):** | 25 mg |
| Rapamycin 42-ester with 3-hydroxy-2-(hydroxymethyl)-2-methylpropionic acid | |
| Polysorbate 80 | 0.5 mL |
| Lactic acid | 0.025 mg |
| **Vial 2 (Diluent):** | |
| Polyethylene glycol 400 | 42.8% w/v |
| Propylene glycol | 33.5% w/v |
| Dehydrated alcohol | 46.23% w/v |

**Table-4 B: Comparative stability profile of the composition that is similar to the marketed composition with the composition of the present invention**

| **Formulation** | **Condition** | **pH** | **Total impurities** |
|---|---|---|---|
| Composition similar to the marketed composition | Initial | 4.40 | 0.93 |
| | 40°C/7Days | 4.30 | 5.35 |
| | 25°C/ 3 Months | 4.36 | 8.76 |
| | 2-8°C/3 Months | 4.43 | 3.31 |
| Formulation of the present invention | Initial | 3.93 | 1.48 |
| | 40°C/7Days | 3.87 | 2.4 |
| | 25°C/ 3 Months | 3.96 | 4.18 |
| | 2-8°C/3 Months | 3.92 | 2.76 |

The comparative stability profile of the pharmaceutical composition prepared from the formulation protocol of the present invention and a formulation that is similar to the marketed composition shows that under stress conditions at 40° C, the impurity profile of the composition of the present invention is substantially lower as compared to the impurity profile of the marketed formulation when both the compositions are stored under similar stress conditions for a period of seven days. Similarly, when the two formulations are stored at 25°C over a period of three months, then the impurities obtained from the composition that is similar to the marketed composition is much higher than the impurities obtained from the composition of the present invention. Similarly, when the two formulations are stored at 2-8°C over a period of three months, then the impurities obtained from the composition that is similar to the marketed composition is much higher than the impurities obtained from the composition of the present invention.

Hence, the above-mentioned pharmaceutical composition of the present invention can be said to be more stable than the composition that is similar to the marketed formulation of Rapamycin 42-ester with 3-hydroxy-2-(hydroxymethyl)-2-methylpropionic acid.

**Example 5:** The pharmaceutical composition provided in this example is a two vial formulation of Rapamycin 42-ester with 3-hydroxy-2-(hydroxymethyl)-2-methylpropionic acid free of any antioxidant. The composition comprises Rapamycin 42-ester with 3-hydroxy-2-(hydroxymethyl)-2-methylpropionic acid dissolved in polysorbate 80(surfactant) along with lactic acid and ethanol in one vial and the second vial contains the diluents which is a mixture of propylene glycol, polyethylene glycol 400 and dehydrated alcohol. The unit composition formula of the pharmaceutical composition prepared by the present inventors is provided in Table-VA.

**Table-5 A: Two Vial Formulation of Rapamycin 42-ester with 3-hydroxy-2-(hydroxymethyl)-2-methylpropionic acid free of antioxidant and containing the drug dissolved in ethanol and a surfactant (polysorbate 80) along with an acid (lactic acid)**

| **Component** | **Quantity per mL** |
|---|---|
| **Vial 1 (Drug Concentrate):** | 25 mg |
| Rapamycin 42-ester with 3-hydroxy-2-(hydroxymethyl)-2-methylpropionic acid | |
| Polysorbate 80 | 0.5 mL |
| Ethanol | q.s. to 1 mL |
| Lactic acid | q.s. to pH 4.0 - 4.5 |
| **Vial 2 (Diluent):** | |
| Polyethylene glycol 400 | 42.8% w/v |
| Propylene glycol | 33.5% w/v |
| Dehydrated alcohol | 46.23% w/v |

**Table- 5 B: Comparative stability profile of the composition that is similar to the marketed composition with the composition of the present invention**

| Formulation | Condition | pH | Total impurities |
|---|---|---|---|
| Formulation similar to the Marketed Composition | Initial | 4.55 | 1.36 |
| | 40°C/3Days | 4.57 | 3.89 |
| | 40°C/7Days | 4.58 | 5.43 |
| Composition of the present invention | Initial | 3.96 | 1.77 |
| | 40°C/3Days | 3.94 | 2.79 |
| | 40°C/7Days | 3.97 | 3.95 |

The comparative stability profile of the pharmaceutical composition prepared from the formulation protocol of the present invention and a formulation that is similar to the marketed composition shows that under stress conditions at 40° C, the impurity profile of the composition of the present invention is substantially lesser than the impurity profile of the marketed formulation when both the compositions are stored under similar stress conditions for a period of three and seven days.

Hence, the abovementioned pharmaceutical composition of the present invention can be said to be more stable than the composition that is similar to the marketed formulation of Rapamycin 42-ester with 3-hydroxy-2-(hydroxymethyl)-2-methylpropionic acid.

**Example 6:** The pharmaceutical composition provided in this example is a single vial formulation of Rapamycin 42-ester with 3-hydroxy-2-(hydroxymethyl)-2-methylpropionic acid free of any antioxidant. The composition comprises Rapamycin 42-ester with 3-hydroxy-2-(hydroxymethyl)-2-methylpropionic acid dissolved in polysorbate 80(surfactant) along with lactic acid , propylene glycol, polyethylene glycol 400 and dehydrated alcohol. The Unit composition formula of the pharmaceutical composition prepared by the present inventors is provided in Table-6 A.

**Table-6 A: Single vial formulation of Rapamycin 42-ester with 3-hydroxy-2-(hydroxymethyl)-2-methylpropionic acid free of any antioxidant and containing an acid (lactic acid)**

| **Component** | **Quantity per mL** |
|---|---|
| **Single Vial:** | 10 mg |
| Rapamycin 42-ester with 3-hydroxy-2-(hydroxymethyl)-2-methylpropionic acid | |
| Propylene glycol | 50.3% w/v |
| Polysorbate 80 | 40% w/v |
| Polyethylene glycol 400 | 42.8% w/v |
| Dehydrated alcohol | 39.5% w/v |
| Lactic acid | q.s. to pH 4.0 - 4.5 |

**Table-6 B: Comparative stability profile of the composition that is similar to the marketed composition with the composition of the present invention**

| **Formulation** | **Condition** | **pH** | **Total impurities** |
|---|---|---|---|
| Composition similar to the marketed composition | Initial | 4.40 | 0.93 |
| | 40°C/7Days | 4.30 | 5.35 |
| | 25°C/ Month | 4.38 | 5.33 |
| | 25°C/2 Months | 4.36 | 7.31 |
| | 25°C/ 3 Months | 4.36 | 8.76 |
| | 25°C/ 6 Months | 4.34 | 9.82 |
| | 2-8°C/ Month | 4.36 | 1.98 |
| | 2-8°C/ 2 Months | 4.40 | 2.79 |
| | 2-8°C/3 Months | 4.43 | 3.31 |
| | 2-8°C/6 Months | 4.46 | 4.02 |
| Formulation of the present invention | Initial | 4.18 | 0.85 |
| | 40°C/ 7Days | 4.15 | 1.89 |
| | 25°C/ 1 Month | 4.12 | 2.84 |
| | 25°C/ 2 Months | 4.12 | 4.17 |
| | 25°C/ 3 Months | 4.15 | 5.55 |
| | 2-8°C/ Month | 4.05 | 1.55 |
| | 2-8°C/2 Months | 4.05 | 2.00 |
| | 2-8°C/3 Months | 4.08 | 2.03 |
| | 2-8°C/ 6 Months | 4.10 | 2.28 |

The comparative stability profile of the pharmaceutical composition prepared from the formulation protocol of the present invention and a formulation that is similar to the marketed composition shows that under stress conditions at 40° C, the impurity profile of the composition of the present invention is substantially less than the impurity profile of the marketed formulation kept under similar storage conditions for a period of seven days. Under other storage conditions such as 25°C for a period of three months and six months, the formulation of the present invention shows a much better stability profile as compared to the formulation of the present invention. Similarly, at 2-8°C the formulation of the present invention shows a better stability profile as compared to the formulation that is similar to the marketed formulation when the two are stored over a period of three months and six months respectively.

Hence, the above-mentioned pharmaceutical composition of the present invention can be said to be more stable than the composition that is similar to the marketed formulation of Rapamycin 42-ester with 3-hydroxy-2-(hydroxymethyl)-2-methylpropionic acid.

**Example 7:** The pharmaceutical composition provided in this example is a single vial formulation of Rapamycin 42-ester with 3-hydroxy-2-(hydroxymethyl)-2-methylpropionic acid free of any antioxidant. The composition comprises Rapamycin 42-ester with 3-hydroxy-2-(hydroxymethyl)-2-methylpropionic acid dissolved in polysorbate 80(surfactant) along with lactic acid and dehydrated alcohol. The unit composition formula of the pharmaceutical composition prepared by the present inventors is provided in Table-7 A.

**Table-7 A: Single vial formulation of Rapamycin 42-ester with 3-hydroxy-2-(hydroxymethyl)-2-methylpropionic acid free of any antioxidant and containing the drug dissolved in a mixture of dehydrated alcohol, polysorbate 80 and acid (lactic acid)**

| **Component** | **Quantity per mL** |
|---|---|
| **Single Vial:** | 10 mg |
| Rapamycin 42-ester with 3-hydroxy-2-(hydroxymethyl)-2-methylpropionic acid | |
| Polysorbate 80 | 40 % w/v |
| Dehydrated alcohol | 39.5% w/v |
| Lactic acid | q.s. to pH 4.0 - 4.5 |

**Table-7 B: Comparative stability profile of the composition that is similar to the marketed composition with the composition of the present invention**

| **Formulation** | **Condition** | **pH** | **Total impurities** |
|---|---|---|---|
| Composition similar to the marketed composition | Initial | 4.4 | 0.93 |
| | 40°C/7Days | 4.30 | 5.35 |
| Formulation of the present invention | Initial | 4.18 | 1.77 |
| | 40°C/7Days | 4.15 | 3.95 |

The comparative stability profile of the pharmaceutical composition prepared from the formulation protocol of the present invention and a formulation that is similar to the marketed composition shows that under stress conditions at 40° C, the impurity profile of the composition of the present invention is substantially less than the impurity profile of the marketed formulation kept under similar storage conditions for a period of seven days.

Hence, the above-mentioned pharmaceutical composition of the present invention can be said to be more stable than the composition that is similar to the marketed formulation of Rapamycin 42-ester with 3-hydroxy-2-(hydroxymethyl)-2-methylpropionic acid .

From the examples as illustrated above, it may be seen that all the pharmaceutical compositions of the present invention as illustrated in the examples above are free of antioxidants and are found to exhibit comparable and in some cases even better stability profile than the currently marketed Torisel™ formulation that contains antioxidants as the key element for providing stability to the formulation.

Hence, all the pharmaceutical compositions of the present invention i.e. single vial as well as two vial formulations are free of antioxidants and show comparable and even better stability profile than a formulation that is similar to the marketed formulation.

## Claims

1. A stable ready-to-use pharmaceutical composition of Rapamycin 42-ester with 3-hydroxy-2-(hydroxymethyl)-2-methylpropionic acid, that is free of antioxidants, comprising a parenterally acceptable solvent, lactic acid and a surfactant.

2. A pharmaceutical composition according to claim 1, which is for parenteral administration.

3. A pharmaceutical composition according to claim 2, wherein the parenteral administration comprises of intravenous, intramuscular or subcutaneous injections.

4. A pharmaceutical composition according to claim 3, wherein the parenteral administration comprises of intravenous injection.

5. A pharmaceutical composition according to claim 1, wherein the Rapamycin 42-ester with 3-hydroxy-2-(hydroxymethyl)-2-methylpropionic acid is present in the range of about 1 mg/ml to about 50 mg/ml.

6. A pharmaceutical composition according to claim 5, wherein the Rapamycin 42-ester with 3-hydroxy-2-(hydroxymethyl)-2-methylpropionic acid is present in the range of about 10 mg/ml to about 25 mg/ml.

7. A pharmaceutical composition according to anyone of claims 5 or 6, wherein the Rapamycin 42-ester with 3-hydroxy-2-(hydroxymethyl)-2-methylpropionic acid is dissolved in the parenterally acceptable solvent.

8. A pharmaceutical composition according to claim 7, wherein the parenterally acceptable solvent is an alcoholic solvent.

9. A pharmaceutical composition according to claim 1, wherein the alcoholic solvent is selected from the group comprising of ethanol, propylene glycol, polyethylene glycol 300, polyethylene glycol 400, and polyethylene glycol 600.

10. A pharmaceutical composition according to claim 1, wherein the surfactant is selected from the group comprising of polysorbate 20, polysorbate 80, a bile acid, lecithin, an ethoxylated vegetable oil, vitamin E tocopherol propylene glycol succinate, or polyoxyethylene-polyoxypropylene block copolymers.

11. A pharmaceutical composition according to claim 10, wherein the surfactant is polysorbate 80.

12. A pharmaceutical composition according to claim 1, having pH in the range of 3.0 to 5.0.

13. A pharmaceutical composition according to claim 12, wherein the pH is preferably in the range of 4.0 to 4.5.

14. A pharmaceutical composition according to claim 1 provided in a single vial, wherein the Rapamycin ester along with the alcoholic solvent, lactic acid and the surfactant are present in one vial.

15. A pharmaceutical composition according to claim 1 provided in two vials, wherein the first vial contains the drug concentrate and the second vial contains the diluents mixture.

16. A process for preparing a stable pharmaceutical composition of Rapamycin 42-ester with 3-hydroxy-2-(hydroxymethyl)-2-methylpropionic acid that is free of antioxidants comprising:
a) maintaining the formulation vessel at a definite temperature;
b) adding a parenterally acceptable solvent, a surfactant in the formulation vessel;
c) adding lactic acid;
d) purging nitrogen gas into the formulation vessel and maintaining nitrogen atmosphere in the headspace;
e) mixing Rapamycin 42-ester with 3-hydroxy-2-(hydroxymethyl)-2-methylpropionic acid with the parenterally acceptable solvent to form the drug concentrate;
f) mixing the parenterally acceptable solvent to form the diluent mixture; and
g) mixing the drug concentrate with the diluent mixture to form the final pharmaceutical composition of Rapamycin 42-ester with 3-hydroxy-2-(hydroxymethyl)-2-methylpropionic acid.

17. The process according to Claim 16, wherein the temperature is maintained at 0-15°C.

18. The process according to Claim 17, wherein the temperature is maintained at 2-8°C.

19. The process according to Claim 17, wherein the parenterally acceptable solvent is an alcoholic solvent.

20. The process according to claim 19, wherein the alcoholic solvent is selected from the group comprising of ethanol, propylene glycol, polyethylene glycol 300, polyethylene glycol 400, and polyethylene glycol 600.

21. The process according to claim 16, wherein the surfactant is selected from the group comprising of polysorbate 20, polysorbate 80, a bile acid, lecithin, an ethoxylated vegetable oil, vitamin E tocopherol propylene glycol succinate, or polyoxyethylene-polyoxypropylene block copolymers.

22. The process according to claim 21, wherein the surfactant is polysorbate 80.

23. The process according to claim 16, wherein Rapamycin 42-ester with 3-hydroxy-2-(hydroxymethyl)-2-methylpropionic acid comprises from about 1 mg/ml to about 50 mg/ml.

24. The process according to claim 23, wherein Rapamycin 42-ester with 3-hydroxy-2-(hydroxymethyl)-2-methylpropionic acid comprises from about 10 mg/ml to about 25 mg/ml.

25. The composition as claimed in claim 1 for use in the treatment of advanced renal cell carcinoma.

## Patentansprüche

1. Stabile, gebrauchsfertige pharmazeutische Zusammensetzung von Rapamycin 42-Ester mit 3-Hydroxy-2-(hydroxymethyl)-2-methylpropionsäure, die frei von Antioxidanzien ist, umfassend ein parenteral akzeptables Lösungsmittel, Milchsäure und ein Tensid.

2. Pharmazeutische Zusammensetzung nach Anspruch 1, die zur parenteralen Verabreichung bestimmt ist.

3. Pharmazeutische Zusammensetzung nach Anspruch 2, wobei die parenterale Verabreichung intravenöse, intramuskuläre oder subkutane Injektionen umfasst.

4. Pharmazeutische Zusammensetzung nach Anspruch 3, wobei die parenterale Verabreichung intravenöse Injektion umfasst.

5. Pharmazeutische Zusammensetzung nach Anspruch 1, wobei der Rapamycin 42-Ester mit 3-Hydroxy-2-(hydroxymethyl)-2-methylpropionsäure im Bereich von ungefähr 1 mg/ml bis ungefähr 50 mg/ml anwesend ist.

6. Pharmazeutische Zusammensetzung nach Anspruch 5, wobei der Rapamycin 42-Ester mit 3-Hydroxy-2-(hydroxymethyl)-2-methylpropionsäure im Bereich von ungefähr 10 mg/ml bis ungefähr 25 mg/ml anwesend ist.

7. Pharmazeutische Zusammensetzung nach einem der Ansprüche 5 oder 6, wobei der Rapamycin 42-Ester mit 3-Hydroxy-2-(hydroxymethyl)-2-methylpropionsäure in dem parenteral akzeptablen Lösungsmittel gelöst ist.

8. Pharmazeutische Zusammensetzung nach Anspruch 7, wobei das parenteral akzeptable Lösungsmittel ein alkoholisches Lösungsmittel ist.

9. Pharmazeutische Zusammensetzung nach Anspruch 1, wobei das alkoholische Lösungsmittel ausgewählt ist aus der Gruppe, bestehend aus Ethanol, Propylenglycol, Polyethylenglycol 300, Polyethylenglycol 400 und Polyethylenglycol 600.

10. Pharmazeutische Zusammensetzung nach Anspruch 1, wobei das Tensid ausgewählt ist aus der Gruppe, bestehend aus Polysorbat 20, Polysorbat 80, einer Gallensäure, Lecithin, einem ethoxylierten Pflanzenöl, Vitamin E Tocopherol-Propylenglycol-Succinat oder Polyoxyethylen-Polyoxypropylen-Block-Copolymeren.

11. Pharmazeutische Zusammensetzung nach Anspruch 10, wobei das Tensid Polysorbat 80 ist.

12. Pharmazeutische Zusammensetzung nach Anspruch 1, mit einem pH im Bereich von 3,0 bis 5,0.

13. Pharmazeutische Zusammensetzung nach Anspruch 12, wobei der PH bevorzugt im Bereich von 4,0 bis 4,5 ist.

14. Pharmazeutische Zusammensetzung nach Anspruch 1, bereitgestellt in einem einzelnen Vial, wobei der Rapamycin-Ester zusammen mit dem alkoholischen Lösungsmittel, Milchsäure und dem Tensid in einem Vial anwesend sind.

15. Pharmazeutische Zusammensetzung nach Anspruch 1, bereitgestellt in 2 Vials, wobei das erste Vial das Arzneimittelkonzentrat enthält und das zweite Vial das Verdünnungsmittelgemisch enthält.

16. Verfahren zur Herstellung einer stabilen pharmazeutischen Zusammensetzung von Rapamycin 42-Ester mit 3-Hydroxy-2-(hydroxymethyl)-2-methylpropionsäure, die frei von Antioxidanzien ist, umfassend:
a) Halten des Formulierungsbehälters bei einer definierten Temperatur ;
b) das Hinzufügen eines parenteral akzeptablen Lösungsmittels und eines Tensids in den Formulierungsbehälter;
c) das Hinzufügen von Milchsäure;
d) das Einleiten von Stickstoffgas in den Formulierungsbehälter und Beibehaltung der Stickstoffatmosphäre in dem Gasraum;
e) das Mischen von Rapamycin 42-Ester mit 3-Hydroxy-2-(hydroxymethyl)-2-methylpropionsäure mit dem pharmazeutisch akzeptablen Lösungsmittel, um das Arzneimittelkonzentrat zu bilden;
f) das Mischen des parenteral akzeptablen Lösungsmittels, um das Verdünnungsmittelgemisch zu bilden; und
g) das Mischen des Arzneimittelkonzentrats mit dem Verdünnungsmittelgemisch, um die endgültige pharmazeutische Zusammensetzung von Rapamycin 42-Ester mit 3-Hydroxy-2-(hydroxymethyl)-2-methylpropionsäure zu bilden.

17. Verfahren nach Anspruch 16, wobei die Temperatur auf 0 - 15 °C gehalten wird.

18. Verfahren nach Anspruch 17, wobei die Temperatur auf 2 -8 °C gehalten wird.

19. Verfahren nach Anspruch 17, wobei das parenteral akzeptable Lösungsmittel ein alkoholisches Lösungsmittel ist.

20. Verfahren nach Anspruch 19, wobei das alkoholische Lösungsmittel ausgewählt ist aus der Gruppe, bestehend aus Ethanol, Propylenglycol, Polyethylenglycol 300, Polyethylenglycol 400 und Polyethylenglycol 600.

21. Verfahren nach Anspruch 16, wobei das Tensid ausgewählt ist aus der Gruppe umfassend Polysorbat 20, Polysorbat 80, eine Gallensäure, Lecithin, ein ethoxyliertenes Pflanzenöl, Vitamin E Tocopherol-Propylenglycol-Succinat oder Polyoxyethylen-Polyoxypropylen-Block-Copolymere.

22. Verfahren nach Anspruch 21, wobei das Tensid Polysorbat 80 ist.

23. Verfahren nach Anspruch 16, wobei Rapamycin 42-Ester mit 3-Hydroxy-2-(hydroxymethyl)-2-methylpropionsäure ungefähr von 1 mg/ml bis ungefähr 50 mg/ml umfasst.

24. Verfahren nach Anspruch 23, wobei Rapamycin 42-Ester mit 3-Hydroxy-2-(hydroxymethyl)-2-methylpropionsäure ungefähr von 10 mg/ml bis ungefähr 25 mg/ml umfasst.

25. Zusammensetzung nach Anspruch 1 zur Verwendung bei der Behandlung von fortgeschrittenem Nierenzellenkarzinom.

## Revendications

1. Composition pharmaceutique prête à l'emploi stable d'ester-42 de rapamycine avec de l'acide 3-hydroxy-2-(hydroxyméthyl)-2-méthylpropionique, qui est exempte d'antioxydants, comprenant un solvant acceptable par voie parentérale, de l'acide lactique et un tensioactif.

2. Composition pharmaceutique selon la revendication 1, qui est destinée à être administrée par voie parentérale.

3. Composition pharmaceutique selon la revendication 2, dans laquelle l'administration par voie parentérale comprend les injections intraveineuses, intramusculaires ou sous-cutanées.

4. Composition pharmaceutique selon la revendication 3, dans laquelle l'administration par voie parentérale comprend une injection intraveineuse.

5. Composition pharmaceutique selon la revendication 1, dans laquelle l'ester-42 de rapamycine avec de l'acide 3-hydroxy-2-(hydroxyméthyl)-2-méthylpropionique est présent à raison d'environ 1 mg/ml à environ 50 mg/ml.

6. Composition pharmaceutique selon la revendication 5, dans laquelle l'ester-42 de rapamycine avec de l'acide 3-hydroxy-2-(hydroxyméthyl)-2-méthylpropionique est présent à raison d'environ 10 mg/ml à environ 25 mg/ml.

7. Composition pharmaceutique selon l'une quelconque des revendications 5 et 6, dans laquelle l'ester-42 de rapamycine avec de l'acide 3-hydroxy-2-(hydroxyméthyl)-2-méthylpropionique est dissous dans le solvant acceptable par voie parentérale.

8. Composition pharmaceutique selon la revendication 7, dans laquelle le solvant acceptable par voie parentérale est un solvant alcoolique.

9. Composition pharmaceutique selon la revendication 1, dans laquelle le solvant alcoolique est choisi dans l'ensemble comprenant l'éthanol, le propylèneglycol, le polyéthylèneglyco1300, le polyéthylèneglycol 400 et le polyéthylèneglycol 600.

10. Composition pharmaceutique selon la revendication 1, dans laquelle le tensioactif est choisi dans l'ensemble comprenant le polysorbate 20, le polysorbate 80, un acide biliaire, la lécithine, une huile végétale éthoxylée, le succinate de propylèneglycol et du tocophérol vitamine E, ou les copolymères séquencés de poly(oxyde d'éthylène)/poly(oxyde de propylène).

11. Composition pharmaceutique selon la revendication 10, dans laquelle le tensioactif est le polysorbate 80.

12. Composition pharmaceutique selon la revendication 1, ayant un pH situé dans la plage allant de 3,0 à 5,0.

13. Composition pharmaceutique selon la revendication 12, dans laquelle le pH est de préférence situé dans la plage allant de 4,0 à 4,5.

14. Composition pharmaceutique selon la revendication 1 fournie dans un flacon unique, dans laquelle l'ester de rapamycine conjointement avec le solvant alcoolique, l'acide lactique et le tensioactif sont présents dans un seul flacon.

15. Composition pharmaceutique selon la revendication 1 fournie dans deux flacons, dans laquelle le premier flacon contient le concentré de médicament et le deuxième flacon contient le mélange de diluants.

16. Procédé pour préparer une composition pharmaceutique stable d'ester-42 de rapamycine avec de l'acide 3-hydroxy-2-(hydroxyméthyl)-2-méthylpropionique, qui est exempte d'antioxydants, comprenant :
a) le maintien du récipient de formulation à une température définie ;
b) l'addition d'un solvant acceptable par voie parentérale dans le récipient de formulation ;
c) l'addition d'acide lactique ;
d) une purge à l'azote gazeux dans le récipient de formulation et le maintien d'une atmosphère d'azote dans l'espace supérieur ;
e) le mélange d'ester-42 de rapamycine avec de l'acide 3-hydroxy-2-(hydroxyméthyl)-2-méthylpropionique avec le solvant acceptable par voie parentérale pour former le concentré de médicament ;
f) le mélange du solvant acceptable par voie parentérale pour former le mélange diluant ; et
g) le mélange du concentré de médicament avec le mélange diluant pour former la composition pharmaceutique finale d'ester-42 de rapamycine avec de l'acide 3-hydroxy-2-(hydroxyméthyl)-2-méthylpropionique.

17. Procédé selon la revendication 16, dans lequel la température est maintenue de 0 à 15 °C.

18. Procédé selon la revendication 17, dans lequel la température est maintenue de 2 à 8 °C.

19. Procédé selon la revendication 17, dans lequel le solvant acceptable par voie parentérale est un solvant alcoolique.

20. Procédé selon la revendication 19, dans lequel le solvant alcoolique est choisi dans l'ensemble comprenant l'éthanol, le propylèneglycol, le polyéthylèneglycol 300, le polyéthylèneglycol 400 et le polyéthylèneglycol 600.

21. Procédé selon la revendication 16, dans lequel le tensioactif est choisi dans l'ensemble comprenant le polysorbate 20, le polysorbate 80, un acide biliaire, la lécithine, une huile végétale éthoxylée, le succinate de propylèneglycol et du tocophérol vitamine E, ou les copolymères séquencés de poly(oxyde d'éthylène)/poly(oxyde de propylène).

22. Procédé selon la revendication 21, dans lequel le tensioactif est le polysorbate 80.

23. Procédé selon la revendication 16, dans lequel l'ester-42 de rapamycine avec de l'acide 3-hydroxy-2-(hydroxyméthyl)-2-méthylpropionique représente d'environ 1 mg/ml à environ 50 mg/ml.

24. Procédé selon la revendication 23, dans lequel l'ester-42 de rapamycine avec de l'acide 3-hydroxy-2-(hydroxyméthyl)-2-méthylpropionique représente d'environ 10 mg/ml à environ 25 mg/ml.

25. Composition selon la revendication 1 pour utilisation dans le traitement d'un carcinome à cellules rénales avancé.
